# EUROPÄISCHE PATENTSCHRIFT

(11) **EP 0 998 883 B1**
(45) Veröffentlichungstag und Bekanntmachung des Hinweises auf die Patenterteilung: **12.09.2007**
(21) Anmeldenummer: 99121828.0
(22) Anmeldetag: 04.11.1999
(51) Int. Cl.: A61F 2/08, A61B 17/88

(54) **Instrumentarium zum Implantieren eines Kreuzbandersatzes in einem Kniegelenk**
Implantation tool for a cruciate ligament in a knee joint
Outil d'implantation pour un ligament croisé dans une articulation du genou

(30) Priorität: 06.11.1998 DE 19851152
(43) Veröffentlichungstag der Anmeldung: 10.05.2000
(73) Patentinhaber: Karl Storz GmbH & Co. KG, 78532 Tuttlingen (DE)
(72) Erfinder: Strobel, Michael, Dr., 94360 Mitterfels (DE); Sauer, Michael, 78532 Tuttlingen (DE)
(74) Vertreter: Weller, Wolfgang

(56) Entgegenhaltungen:
- EP-A- 0 304 107
- WO-A-97/18762
- DE-A- 19 503 504
- US-A- 3 538 916
- US-A- 5 176 682
- US-A- 5 797 918

## Beschreibung

Die vorliegende Erfindung betrifft ein Instrumentarium zum Implantieren eines Kreuzbandersatzes in einem Kniegelenk.

Aus der WO 97/18762 A ist ein Instrumentarium bekannt, das einen Applikator zum Einsetzen eines Dübels in eine Bohrung aufweist, wobei der Applikator einen an seinem distalen Ende offenen Hohlschaft sowie einen in dem Hohlschaft geführten Stößel aufweist, und wobei der Applikator ein Bedienelement besitzt, mit dem der Stößel derart in dem Hohlschaft verschiebbar ist, dass ein in den Hohlschaft eingebrachter Dübel durch die Öffnung an dem distalen Ende des Hohlschaftes ausstoßbar ist.

Aus der EP-A-0 304 107 ist eine Vorrichtung zum Injizieren eines Implantates in ein Weichgewebe bekannt. Das Implantat enthält ein Arzneimittel, das nach Injizieren des Implantates im Weichgewebe freigesetzt werden soll. Die Vorrichtung zum Injizieren des Implantates weist einen als Spitze ausgebildeten Hohlschaft auf, in den das Implantat völlig eingebracht werden kann. In dem Hohlschaft ist ein Stößel aufgenommen, der über ein Bedienelement bewegbar ist. Der Stößel ist derart im Hohlschaft verschiebbar, dass ein im Hohlschaft eingebrachtes Implantat durch die Öffnung am distalen Ende ausstoßbar ist. Ferner ist ein lösbarer Verriegelungsmechanismus vorgesehen, um den Stößel relativ zu dem Hohlschaft festzustellen.

Aus der US-A-3 538 916 ist eine Injektionspistole bekannt, um intramuskuläre Implantate einzusetzen. In einem angespitzten Hohlschaft ist ein Stößel verschieblich aufgenommen, der über ein Bedienelement im Schaft hin und her verschiebbar ist.

Operationen, bei denen ein Kreuzbandersatz in einem Kniegelenk implantiert wird, sind im Stand der Technik grundsätzlich bekannt. Solche Operationen werden erforderlich, wenn das Kreuzband im Knie gerissen oder anderweitig stark beschädigt ist. Als Kreuzbandersatz werden dabei heutzutage bevorzugt körpereigene Materialien verwendet. Dabei haben sich insbesondere die Patellar-Sehne oder die Semitendinosus-Sehne als geeignet erwiesen, den benötigten Kreuzbandersatz herzustellen.

Ein Verfahren und ein Instrumentarium zur Rekonstruktion des vorderen Kreuzbandes mit Hilfe einer dieser Sehnen als Kreuzbandersatz ist aus der EP-A-0 440 991 bekannt.

Bei diesem bekannten Verfahren wird in dem Fall, daß die Semitendinosus-Sehne als Kreuzbandersatz verwendet werden soll, diese zunächst mit Hilfe eines Sehnenschneiders entfernt und anschließend in einzelne Segmente oder Abschnitte unterteilt. Manche dieser Segmente werden in Form einer Schlaufe zusammengelegt, wodurch sich Doppelsegmente ergeben. Die einzelnen Segmente und/oder die Doppelsegmente werden anschließend wiederum nebeneinander gelegt und an ihren Enden mit Nahtfäden miteinander verbunden. Das auf diese Weise hergestellte Sehnenbündel bildet dann den Kreuzbandersatz.

Dieser Kreuzbandersatz wird dann zwischen dem distalen Ende des Femur und dem proximalen Ende der Tibia befestigt. Hierzu wird zunächst im proximalen Ende der Tibia eine Durchgangsbohrung und im distalen Ende des Femur eine Sacklochbohrung gebohrt. In diese beiden Bohrungen wird dann jeweils ein Ende des Kreuzbandersatzes eingesetzt und in den Bohrungen fixiert.

Die Fixierung des Kreuzbandersatzes in der Durchgangsbohrung der Tibia erfolgt gemäß der EP-A-0 440 991 mit Hilfe einer Schraube. Demgegenüber wird der Kreuzbandersatz in der Sacklochbohrung des Femur fixiert, indem er mit Nahtfäden, die durch einen dünnen, sich an die Sacklochbohrung anschließenden Bohrkanal geführt sind, und einer knopfartigen Platte an der der Sacklochbohrung gegenüberliegenden Außenseite des Femur verspannt wird.

Diese rein mechanische Befestigung des Kreuzbandersatzes in den beiden Bohrungen wird bei einer erfolgreich verlaufenden Operation im Laufe der Zeit durch ein Anwachsen des Kreuzbandersatzes an dem ihn umgebenden Knochenmaterial verstärkt. Erst dieses Anwachsen gewährleistet einen dauerhaften und zuverlässigen Halt des Kreuzbandersatzes im Knie.

Es hat sich jedoch nun gezeigt, daß der Kreuzbandersatz bei diesem bekannten Verfahren nicht in allen Fällen wie gewünscht mit dem ihn umgebenden Knochenmaterial verwächst. Dies tritt insbesondere dann auf, wenn der Kreuzbandersatz in der ihn aufnehmenden Bohrung zu locker sitzt. In diesem Fall bewegt sich der Kreuzbandersatz nämlich auch bei kleinen Bewegungen des Knies, so daß eine sich erst bildende Verwachsung leicht wieder aufgebrochen wird. Eine zweite, ebenfalls häufig auftretende Ursache für diese Schwierigkeiten ist, daß der Kreuzbandersatz selbst zu wenig Kontakt mit dem ihn umgebenden Knochenmaterial bekommt.

Letzteres tritt insbesondere dann auf, wenn der Kreuzbandersatz, wie bereits erwähnt, aus in Form einer Schlaufe zusammengelegten Segmenten besteht. In diesem Fall ist nämlich aufgrund der maximalen Biegeradien das Kopfende des Kreuzbandersatzes, d.h. der Bereich der Schlaufe breiter als der Bereich unterhalb davon. Wenn nun die Schlaufe wie üblich in die Sacklochbohrung des Femur eingesetzt werden soll, bestimmt die Breite der Schlaufe die Größe bzw. den Durchmesser der Sacklochbohrung. Dies führt jedoch dazu, daß zwischen dem Kreuzbandersatz unterhalb der Schlaufe und den Wänden der Sacklochbohrung Freiräume verbleiben. Infolge dieser Freiräume besitzt der Kreuzbandersatz an diesen Stellen keinen Kontakt mit dem ihn umgebenden Knochenmaterial, wodurch ein Anwachsen erschwert oder sogar verhindert wird.

Der vorliegenden Erfindung liegt von daher der Gedanke zugrunde, die genannten Schwierigkeiten zu vermeiden. Dies kann auf einfache und sehr erfolgreiche Weise dadurch geschehen, daß in die Freiräume zwischen dem Kreuzbandersatz und den Wänden der ihn aufnehmenden Bohrung Dübel eingebracht werden, die den Kreuzbandersatz einerseits in der Bohrung fixieren und andererseits den Kontakt zwischen dem Kreuzbandersatz und den Wänden der Bohrung verbessern oder sogar erst herstellen. Eine derartige Ergänzung des zuvor beschriebenen Operationsverfahrens ist im Stand der Technik bisher nicht bekannt.

Das Einsetzen solcher Dübel in eine Bohrung, in die bereits ein Kreuzbandersatz implantiert ist, erfordert jedoch, wie angesichts der sehr geringen Platzverhältnisse und Zugangsmöglichkeiten in einem Kniegelenk leicht einzusehen ist, ein geeignetes und am besten sogar ein speziell darauf angepaßtes Instrumentarium. Ein derartiges Instrumentarium ist aus dem Stand der Technik jedoch ebenfalls nicht bekannt.

Es ist von daher Aufgabe der Erfindung, ein Instrumentarium zum Implantieren eines Kreuzbandersatzes in einem Kniegelenk anzugeben, mit dem ein oder mehrere Dübel in Freiräume zwischen dem Kreuzbandersatz und den Wänden der ihn aufnehmenden Bohrung eingesetzt werden kann.

Die Aufgabe wird erfindungsgemäß durch ein Instrumentariums zum Implantierten eines Kreuzbandersatzes in einem Kniegelenk gelöst, das einen Applikator zum Einsetzen eines Dübels in eine Bohrung aufweist, in der der Kreuzbandersatz aufgenommen ist, und zwar in einen Freiraum zwischen dem Kreuzbandersatz und einer Wand in der Bohrung, an der der Kreuzbandersatz fixiert werden soll, wobei der Applikator einen an seinem distalen Ende offenen Hohlschaft sowie einen in dem Hohlschaft geführten Stößel aufweist, und wobei der Applikator ein Bedienelement besitzt, mit dem der Stößel derart in dem Hohlschaft verschiebbar ist, dass ein in den Hohlschaft eingebrachter Dübel durch die Öffnung an dem distalen Ende des Hohlschaftes ausstoßbar ist, wobei der Applikator einen lösbaren Verriegelungsmechanismus zum Feststellen des Stößels relativ zu dem Hohlschaft aufweist, wobei das Instrumentarium außerdem mehrere Messstäbe zum Bestimmen der Größe des Freiraumes aufweist, wobei das distale Ende jedes Messstabes in etwa der Form des einzusetzenden Dübels entspricht und wobei die Enddurchmesser D_{M} der Messstäbe voneinander verschieden sind, und es weiterhin zumindest einen Stopfer beinhaltet, mit dem der Dübel in den Freiraum hineinpressbar ist.

Das distale Ende ist dabei der üblichen Fachterminologie entsprechend das von dem Bedienelement entfernt liegende Ende des Hohlschaftes.

Mit einem derartigen Applikator ist es möglich, die beiden zum Einsetzen eines Dübels in den genannten Freiraum notwendigen Schritte, nämlich das Heranbringen des Dübels an den Freiraum und das Einschieben des Dübels in den Freiraum, kontrolliert und sicher durchzuführen. Dies gilt insbesondere für die heutzutage bei Knieoperationen üblichen arthroskopischen Eingriffe.

Bei der Anwendung des Applikators wird der einzusetzende Dübel zunächst also in den Hohlschaft eingebracht. Daran anschließend wird das distale Ende des Hohlschaftes an den Freiraum herangeführt, in den der Dübel eingesetzt werden soll. Erst wenn sich die Öffnung am distalen Ende des Hohlschaftes in einer geeigneten Position an oder vor dem Freiraum befindet, wird der Stößel innerhalb des Hohlschaftes mit Hilfe des Bedienelementes in Richtung des distalen Endes vorgeschoben. Hierdurch ist es dann möglich, den in dem Hohlschaft befindlichen Dübel gezielt und kontrolliert in den Freiraum zwischen dem Kreuzbandersatz und der ihn umgebenden Wand der Bohrung einzusetzen.

Das Heranbringen des Dübels an den Freiraum mit Hilfe des ihn aufnehmenden Hohlschaftes bietet den Vorteil, daß der Dübel auch bei den im Kniegelenk vorherrschenden, beengten Platzverhältnissen sicher und gezielt geführt werden kann. Im Vergleich beispielsweise zu einer Pinzette oder einem ähnlichen Instrument ist somit gewährleistet, daß der Dübel im Kniegelenk nicht durch eine unachtsame Bewegung oder ein Anstoßen an einem Knochen verlorengehen kann.

Des weiteren bietet das Ausstoßen des Dübels aus dem Hohlschaft mit Hilfe eines über ein Bedienelement verschiebbaren Stößels den Vorteil, daß auf den Dübel bereits beim Einsetzen in den Freiraum ein gezielter Druck von hinten ausgeübt werden kann, der notwendig ist, um den Dübel möglichst fest zwischen dem Kreuzbandersatz und den Wänden der Bohrung zu verkeilen.

Insgesamt ist somit ein gezieltes und kontrolliertes Einsetzen des Dübels trotz der beengten Platzverhältnisse und der eingeschränkten Zugänglichkeit zu dem genannten Freiraum möglich. Dementsprechend wird die gestellte Aufgabe durch das genannte Instrumentarium vollständig gelöst.

In einer Ausgestaltung der Erfindung ist der Hohlschaft gekrümmt .

Diese Maßnahme besitzt den Vorteil, daß die Spitze des Hohlschaftes hierdurch einfacher an die den Kreuzbandersatz aufnehmende Bohrung und damit an den zu füllenden Freiraum herangeführt werden kann. Die Bohrung mündet nämlich an der Gelenkfläche des Femur, die der Gelenkfläche der Tibia gegenüberliegt. Aufgrund der genannten Maßnahme kann man mit dem gekrümmten Hohlschaft des Applikators seitlich an die Mündungsöffnung herantreten.

In einer weiteren Ausgestaltung der zuvor genannten Maßnahme ist der Hohlschaft über seine Länge gleichmäßig gekrümmt.

Diese Maßnahme besitzt den Vorteil, daß aufgrund der Gleichmäßigkeit der Krümmung der Stößel innerhalb des Hohlschaftes einfach und vor allem ruckfrei verschoben werden kann. Dies ist zum kontrollierten Einsetzen des Dübel vorteilhaft.

In einer alternativen Ausgestaltung der zuvor genannten Maßnahme ist der Hohlschaft entlang zumindest seines Mittelabschnitts im wesentlichen gerade und er ist in der Nähe seines distalen Endes in eine Richtung abgebogen.

Diese Maßnahme besitzt den Vorteil, daß hierbei zwar eine Krümmung vorhanden ist, die das Heranführen des Applikators an den Freiraum erleichtert, daß dabei jedoch gleichzeitig die Sicht des Operateurs auf den Freiraum durch das Instrument weniger behindert ist.

In einer weiteren alternativen Ausgestaltung ist der Hohlschaft durchgehend gerade.

Diese Maßnahme besitzt den Vorteil, daß der Stößel hierdurch ohne jede Behinderung in dem Hohlschaft verschoben werden kann. Dies bedeutet, daß dem Stößel nur ein sehr geringer Reibungswiderstand entgegensteht, was die Bedienbarkeit des gesamten Applikators erleichtert.

In einer Ausgestaltung der zuvor genannten Maßnahmen weist der Stößel eine Verdickung an seinem distalen Ende sowie einen dünneren Mittelabschnitt der Länge L₃ auf, wobei der Außendurchmesser Dᵥ der Verdickung dem lichten Innendurchmesser D_{A} des Hohlschaftes entspricht und wobei der Außendurchmesser D_{St} des Mittelabschnitts geringer ist als der Außendurchmesser Dᵥ der Verdickung.

Diese Maßnahme besitzt den Vorteil, daß der Stößel aufgrund seines dünneren Mittelabschnitts sehr flexibel ist und somit leichter der Krümmung des Hohlschaftes folgen kann. Hierdurch wird erreicht, daß der Stößel innerhalb des Hohlschaftes noch leichter und ruckfreier verschoben werden kann. Gleichzeitig besitzt die Maßnahme aufgrund der Verdickung am distalen Ende des Stößels jedoch den Vorteil, daß auf einen in den Hohlschaft eingebrachten Dübel ein den gesamten Hohlschaft ausfüllendes Stoßelement einwirkt, wodurch der Dübel gleichmäßiger und kontrollierter geführt ist und sich nicht im Hohlschaft verkanten oder verklemmen kann. Insgesamt wird durch die genannt Maßnahme somit das kontrollierte Ausstoßen des Dübels aus dem Hohlschaft verbessert.

In einer weiteren Ausgestaltung der Erfindung ist das distale Ende des Stößels zwischen einer ersten und einen zweiten Position verschiebbar, wobei sich das distale Ende in der ersten Position vollständig innerhalb des Hohlschaftes befindet, und zwar in einem Abstand S₁ zu der Öffnung des Hohlschaftes, und wobei das distale Ende in der zweiten Position über die Öffnung des Hohlschaftes hinausragt.

Diese Maßnahme besitzt den Vorteil, daß der Dübel sicher innerhalb des Hohlschaftes gehalten werden kann, solange sich der Stößel in der ersten Position befindet, und daß der Dübel beim Ausstoßen vollständig, d.h. über seine gesamte Länge, aus dem Hohlschaft herausgedrückt werden kann. Hierdurch ist gewährleistet, daß der Dübel nach dem Ausstoßen und beim Zurücknehmen des Applikators nicht unbeabsichtigt in dem Freiraum verschoben wird oder sogar vollständig aus diesem wieder mit herausgerissen wird. Letzteres könnte insbesondere dann auftreten, wenn der Dübel beispielsweise aufgrund von Gewebeteilchen oder aufgrund von Adhäsionskräften an dem Material des Hohlschaftes haften bleibt. Darüber hinau besitzt die Maßnahme den Vorteil, daß der Dübel somit bereits beim Einsetzen in den Freiraum sehr tief und mit ausreichendem Druck in diesen hineingestoßen werden kann.

In einer weiteren Ausgestaltung der Erfindung ist das Bedienelement entlang einer Bedienachse bedienbar, und der Öffnungsquerschnitt der Öffnung des Hohlschaftes ist in bezug auf die Bedienachse in einem Winkel α von weniger als 90°, vorzugsweise zwischen 0° und 45° angeordnet.

Diese Maßnahme ist insbesondere im Hinblick darauf zu sehen, daß das Einsetzen eines Dübels in die Sacklochbohrung im Femur erfolgt. Dies bedeutet, daß der Dübel von unten oder zumindest von schräg unten an den ihn aufnehmenden Freiraum herangebracht werden muß. Gleichzeitig ist die Unterseite des Femur jedoch nur begrenzt zugänglich. Die genannte Maßnahme beinhaltet anschaulich gesprochen, daß die Austrittsrichtung des Dübels aus dem Hohlschaft gegenüber der Bedienachse des Applikators gekippt ist, und zwar in dem besonders bevorzugten Fall im rechten Winkel. Hierdurch wird das Heranbringen des Dübels an den ihn aufnehmenden Freiraum in vorteilhafter Weise erleichtert.

In einer weiteren Ausgestaltung der Erfindung weist das Bedienelement des Applikators einen Griff am proximalen Ende des Stößels, vorzugsweise einen mit dem Daumen bedienbaren Ringgriff, sowie einen Gegengriff an dem Hohlschaft auf.

Das proximale Ende des Stößels ist dabei der üblichen Terminologie entsprechend dasjenige Ende des Stößels, das zu dem distalen Ende des Hohlschaftes entgegengesetzt liegt. Die Maßnahme besitzt den Vorteil, daß der Operateur somit direkt an dem Stößel angreifen kann, um den Dübel aus dem Hohlschaft herauszuschieben. Hierdurch erhält er ein besseres Gefühl für den Dübel als in dem Fall, daß der Stößel erst durch weitere Verbindungselemente mit dem Bedienelement verbunden wäre. Der Gegengriff an dem Hohlschaft bietet in Kombination mit dem Griff am proximalen Ende des Stößels den Vorteil, daß der Applikator dadurch mit einer Hand leicht zu bedienen ist. Die bevorzugte Ausgestaltung des Griffs in Form eines Ringgriffs bietet den Vorteil, daß der Operateur den Stößel ohne ein ansonsten notwendiges Umgreifen auch in entgegengesetzter Richtung bewegen kann, d.h. also den Stößel auch zurückziehen kann, beispielsweise, um die Position des Dübels in dem ihn aufnehmenden Freiraum zu korrigieren. Darüber hinaus sind die genannten Maßnahmen konstruktiv sehr einfach, und ein entsprechender Applikator ist somit kostengünstig herzustellen. Des weiteren ermöglicht die vergleichsweise einfache Konstruktion eine gute Sterilisierbarkeit des Applikators im Anschluß an seine Verwendung.

In einer weiteren Ausgestaltung der Erfindung ist der Stößel vollständig aus dem Hohlschaft herausziehbar.

Diese Maßnahme dient ebenfalls einer einfachen und gründlichen Sterilisierung des Applikators und besitzt den Vorteil, daß somit sowohl der Hohlschaft als auch der in ihm geführte Stößel beim Sterilisieren gut zugänglich sind.

Entsprechend der Erfindung weist der Applikator einen lösbaren Verriegelungsmechanismus zum Feststellen des Stößels relativ zu dem Hohlschaft auf.

Diese Maßnahme besitzt insbesondere in Kombination mit der zuvor genannten Maßnahme den Vorteil, daß der Stößel mit dem Hohlschaft verrastbar ist und somit ein unbeabsichtigtes Herausfallen verhindert wird. Die Maßnahme besitzt jedoch auch für sich genommen den Vorteil, daß der Stößel in der Phase, in der ein in dem Hohlschaft aufgenommener Dübel an den Freiraum herangebracht werden soll, nicht unbeabsichtigt verschoben werden kann, wodurch der Dübel unbeabsichtigt aus dem Hohlschaft herausgestoßen werden könnte. Die Maßnahme trägt somit zur Vermeidung von Fehlern oder Mißgeschicken mit unter Umständen schwerwiegenden Folgen bei.

In einer Ausgestaltung der zuvor genannten Maßnahme weist der Verriegelungsmechanismus einen mit einer Feder vorgespannten Hebel auf, an dessen Ende ein Zapfen angeordnet ist, der im verriegelten Zustand in eine Ringnut des Stößels greift.

Ein derartiger Verriegelungsmechanismus ist von seiner Konstruktion her einerseits sehr einfach und gewährleistet andererseits jedoch eine ausreichend zuverlässige Funktion. Der einfache Aufbau des Verriegelungsmechanismus trägt wiederum zu einer einfachen und gründlichen Sterilisierbarkeit bei. Gleichzeitig werden jedoch auch die Kosten des Applikators bei der Herstellung reduziert. Darüber hinaus ist ein derartiger Verriegelungsmechanismus vom Operateur auch sehr einfach zu bedienen, was wiederum zur Vermeidung von Fehlern bei der Operation vorteilhaft ist.

In einer weiteren Ausgestaltung der Erfindung beinhaltet das Instrumentarium mehrere Applikatoren, deren Innendurchmesser T_{A} voneinander verschieden sind.

Diese Maßnahme besitzt den Vorteil, daß für unterschiedlich große zu setzende Dübel ein jeweils exakt angepaßter und damit optimal geeigneter Applikator zur Verfügung steht. Die unterschiedlichen Dübelgrößen ergeben sich, wie nachfolgend noch ausführlicher erläutert wird, daraus, daß die einzusetzenden Dübel vorteilhafterweise jeweils optimal an die sie aufnehmenden Freiräume und die dort herrschenden Platzverhältnisse angepaßt werden.

Entsprechend der Erfindung weist das Instrumentarium mehrere Meßstäbe zum Bestimmen der Größe des Freiraums auf, wobei das distale Ende jedes Meßstabes in etwa der Form des einzusetzenden Dübels entspricht und wobei die Enddurchmesser D_{M} der Meßstäbe voneinander verschieden sind.

Die Bestimmung der Größe des Freiraums, in die der Dübel eingesetzt werden soll, erfolgt mit diesen Meßstäben auf einfache Weise dadurch, daß zunächst das distale Ende eines der Meßstäbe in den Freiraum eingeführt wird. Anhand des Enddurchmessers D_{M} dieses Meßstabes erhält der Operateur dann ein Vergleichsmaß für die Größe des Freiraums. Eine genauere Bestimmung der Größe des Freiraums ist möglich, indem weitere Meßstäbe mit anderen Enddurchmessern D_{M} in den auszumessenden Freiraum eingeschoben werden. Der Enddurchmesser D_{M} desjenigen Meßstabes, der am besten in den Freiraum hineinpaßt, ist ein Maß für den benötigten Durchmesser des einzusetzenden Dübels.

Die Ergänzung des erfindungsgemäßen Instrumentariums durch derartige Meßstäbe besitzt somit den Vorteil, daß hierdurch die Bestimmung der Größe des Freiraums und damit der Größe des benötigten Dübels auf sehr einfache und trotzdem genaue Weise möglich ist. Dies gilt insbesondere auch im Hinblick darauf, daß der Dübel mit einem gewissen Druck in den Freiraum zwischen dem Kreuzbandersatz und den Wänden der Bohrung eingeschoben werden muß, um den Kreuzbandersatz in der Bohrung so fest wie möglich zu verkeilen. Da der Kreuzbandersatz selbst jedoch eine gewisse Elastizität aufweist, wird der Freiraum unter dem Druck zusätzlich aufgeweitet. Dies wird bei der Bestimmung der Größe des Freiraums mit Hilfe der genannten Meßstäbe automatisch berücksichtigt, da auch diese mit dem erforderlichen Druck in den Freiraum eingeführt werden können. Darüber hinaus sind die erfindungsgemäßen Meßstäbe auch einfach und gründlich zu sterilisieren.

In einer Ausgestaltung der zuvor genannten Maßnahme weist jeder Meßstab an seinem distalen Ende zumindest eine sichtbare Markierung auf, die in einem vorbestimmten Abstand S₃ zum Ende des Meßstabes angeordnet ist.

Diese Maßnahme besitzt den Vorteil, daß somit nicht nur der Durchmesser des einzusetzenden Dübels, sondern auch seine Länge bestimmbar ist. Die sichtbare Markierung kann beim Einschieben des Meßstabes in den Freiraum anhand der bei arthroskopischen Eingriffen üblicherweise verwendeten Kamera oder eines Endoskopes gut beobachtet werden.

Entsprechend der Erfindung beinhaltet das Instrumentarium weiterhin zumindest einen Stopfer, mit dem der Dübel in den Freiraum hineinpreßbar ist.

Diese Maßnahme besitzt den Vorteil, daß der Dübel, nachdem er mit Hilfe des Applikators in den Freiraum eingesetzt worden ist, nun weiter mit einem wesentlich höheren Druck in dem Freiraum verkeilt werden kann. Der höhere Druck, der mit dem Stopfer im Vergleich zu dem Applikator aufgebracht werden kann, resultiert vor allem daraus, daß der Stößel des Applikators aus den zuvor erläuterten Gründen eine gewisse Flexibilität besitzt. Diese wird jedoch bei dem Stopfer nicht benötigt, so daß mit dem Stopfer erheblich mehr Kraft auf den Dübel aufgebracht werden kann.

In einer weiteren Ausgestaltung der zuvor genannten Maßnahme weist der Stopfer an seinem distalen Ende eine konkav gewölbte Stoßfläche auf.

Diese Maßnahme besitzt den Vorteil, daß sich der Dübel beim Hineinpressen in den Freiraum automatisch in bezug auf die Stoßfläche des Stopfers zentriert, so daß die Kraft mit Hilfe des Stopfers optimal auf ihn übertragen wird. Darüber hinaus wird auch das Risiko, daß der Stopfer beim Hineinpressen des Dübels an diesem abrutscht, verringert.

In einer weiteren Ausgestaltung der beiden zuvor genannten Maßnahmen weist der Stopfer an seinem distalen Ende in einer vorbestimmten Entfernung S₂ zur Stoßfläche eine sichtbare Markierung auf.

Diese Maßnahme besitzt den Vorteil, daß anhand der Markierung leichter feststellbar ist, wie weit der Dübel in den zuvor mit Hilfe des Meßstabes bzw. der Meßstäbe ausgemessenen Freiraums eingeschoben ist. Hierdurch ist es möglich, zu entscheiden, ob der Dübel noch tiefer in den Freiraum hineingepreßt werden muß oder ob er sich bereits in der beabsichtigten Endstellung befindet.

In einer weiteren Ausgestaltung der zuvor genannten Maßnahmen beinhaltet das Instrumentarium mehrere Stopfer, deren Enddurchmesser D_{S} voneinander verschieden sind.

Diese Maßnahme besitzt den Vorteil, daß somit für unterschiedlich große Dübel und die damit verbundenen unterschiedlichen Platzverhältnisse jeweils optimal angepaßte Stopfer zur Verfügung stehen. Hierdurch ist es möglich, die Dübel unabhängig von ihrer Größe optimal in den Freiraum zwischen dem Kreuzbandersatz und den Wänden der ihn aufnehmenden Bohrung zu verkeilen.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine schematische Querschnittsansicht eines Kniegelenks mit einem Kreuzbandersatz;
- Fig. 2-5: einzelne Verfahrensschritte beim Herstellen eines Kreuzbandersatzes aus der Semitendinosus-Sehne;
- Fig. 6: eine Draufsicht auf eine Platte zum Fixieren des Kreuzbandersatzes im Femur;
- Fig. 7: eine Querschnittsansicht der Platte aus Fig. 6 entlang der Schnittlinie VII-VII;
- Fig. 8: eine weitere Platte zum Fixieren des Kreuzbandersatzes an der Tibia in einer Draufsicht;
- Fig. 9: eine Querschnittsansicht der Platte aus Fig. 8 entlang der Schnittlinie IX-IX;
- Fig. 10: eine Querschnittsansicht des im Femur eingesetzten Kreuzbandersatzes entlang der Schnittlinie X-X in Fig. 1;
- Fig. 11: ein bevorzugtes Ausführungsbeispiel des erfindungsgemäßen Applikators in einer teilweise aufgeschnittenen Darstellung;
- Fig. 12: den Stößel des erfindungsgemäßen Applikators aus Fig. 11;
- Fig. 13: den Applikator aus Fig. 11 in einer zweiten Betriebsstellung;
- Fig. 14: ein weiteres bevorzugtes Ausführungsbeispiel eines erfindungsgemäßen Applikators;
- Fig. 15: den Stößel des Applikators gemäß Fig. 14;
- Fig. 16: den Hohlschaft des Applikators gemäß Fig. 14;
- Fig. 17: ein bevorzugtes Ausführungsbeispiel des erfindungsgemäßen Stopfers;
- Fig. 18: eine Kompressionszange in einer Seitenansicht;
- Fig. 19: die Kompressionszange aus Fig. 18 in einer Draufsicht;
- Fig. 20: ein bevorzugtes Ausführungsbeispiel eines erfindungsgemäßen Meßstabes;
- Fig. 21: die Verwendung des Meßstabes aus Fig. 20 zum Bestimmen der Größe des Freiraums zwischen dem Kreuzbandersatz und der Wand der ihn aufnehmenden Bohrung im Femur; und
- Fig. 22: die Anwendung des erfindungsgemäßen Stopfers aus Fig. 17.

Wie anhand dieser Figurenbeschreibung ersichtlich ist, werden zur eingehenden Erläuterung des erfindungsgemäßen Instrumentariums im folgenden auch einzelne Schritte des der Erfindung zugrunde liegenden Verfahrens dargestellt.

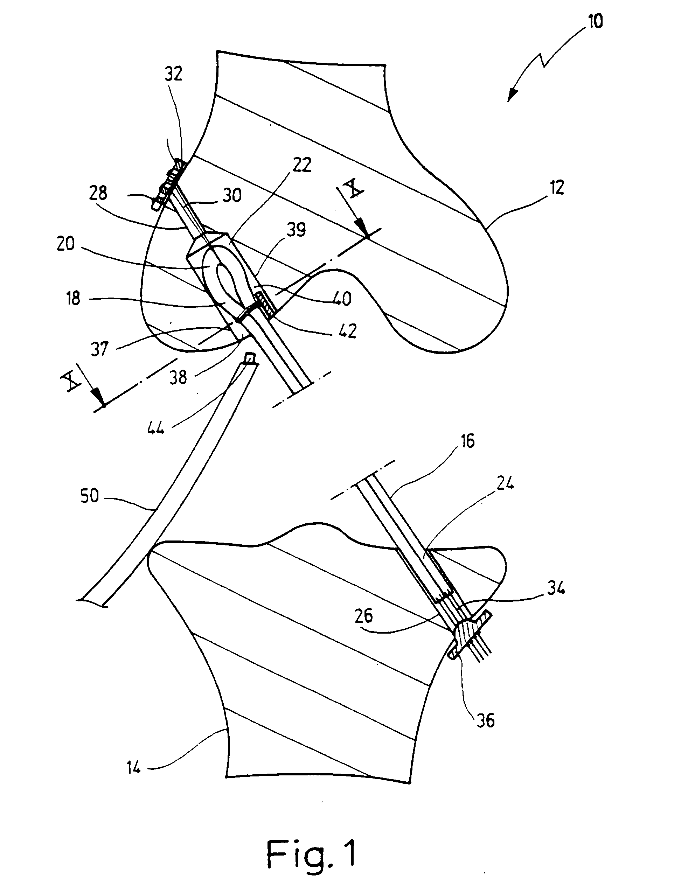

In Fig. 1 ist ein schematisch skizziertes Kniegelenk in seiner Gesamtheit mit der Bezugsziffer 10 bezeichnet.

Das Kniegelenk 10 bildet die Verbindung zwischen dem distalen Ende des Femur 12 und dem proximalen Ende der Tibia 14. Zwischen den beiden, die aus Gründen der Übersichtlichkeit in einem nicht-maßstabsgetreuen Abstand zueinander dargestellt sind, befindet sich ein Kreuzbandersatz 16. Das proximale Ende 18 des Kreuzbandersatzes 16 liegt in einer Schlaufe 20. Die Schlaufe 20 ist zur Befestigung des Kreuzbandersatzes 16 am Femur 12 in eine Sacklochbohrung 22 eingesetzt.

Das distale Ende 24 des Kreuzbandersatzes 16 befindet sich in einer Durchgangsbohrung 26, die in das proximale Ende der Tibia eingebracht ist.

An das Ende der Sacklochbohrung 22 im Femur 12 schließt sich ein dünner Bohrkanal 28 an, durch den ein Faden 30 geführt ist. Der Faden 30 ist mit Hilfe einer Platte 32 an der der Sacklochbohrung 22 gegenüberliegenden Außenseite des Femur 12 verspannt und übt eine Zugkraft auf die Schlaufe 20 des Kreuzbandersatzes 16 aus. Hierdurch wird der Kreuzbandersatz 16 in dem distalen Ende des Femur 12 fixiert.

Das distale Ende 24 des Kreuzbandersatzes 16 ist in vergleichbarer Weise mit Hilfe eines oder mehrerer Fäden 34 sowie einer Platte 36 in der Durchgangsbohrung 26 im proximalen Ende der Tibia 14 verspannt. Die Fäden 30, 34 sind dabei im Stand der Technik hinlänglich bekannte und üblicherweise verwendete Nahtfäden.

Unterhalb des Kopfbereiches der Schlaufe 20 verbleibt zwischen dem Kreuzbandersatz 16 und der Wand 37 der Sacklochbohrung 22 ein Freiraum 38. Dieser Freiraum 38 ergibt sich zwangsläufig daraus, daß die Schlaufe 20 in ihrem Kopfbereich mehr Platz benötigt als unterhalb ihres Kopfbereiches. Zwischen der Wand 39 der Sacklochbohrung 22 und dem Kreuzbandersatz 16 verbleibt ebenso ein Freiraum 40.

Gemäß dem der Erfindung zugrunde liegenden Verfahren werden in die Freiräume 38, 40 Dübel 42 bzw. 44 eingesetzt. Die Dübel 42, 44 sind in diesem Fall Dübel aus körpereigenem Knochenmaterial, nämlich aus dem Knochenschwamm, der beim Bohren der Sacklochbohrung 22 und der Durchgangsbohrung 26 anfällt. In der Darstellung der Fig. 1 befindet sich der Dübel 42 bereits in dem Freiraum 40, während der Dübel 44 gerade mit Hilfe eines bevorzugten Applikators 50 in den Freiraum 38 eingesetzt wird.

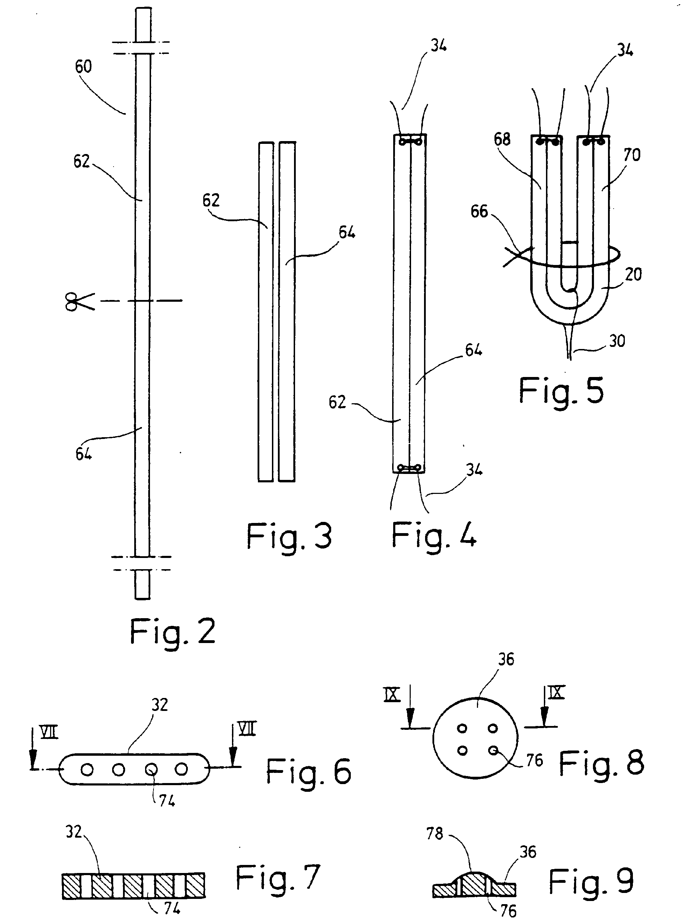

Das Zusammenlegen des Kreuzbandersatzes 16 in Form der Schlaufe 20 geschieht üblicherweise, wenn der Kreuzbandersatz 16 aus der Semitendinosus-Sehne hergestellt wird. Dazu wird zunächst zumindest ein Teil der Semitendinosus-Sehne, häufig das mittlere Drittel, entfernt. Gemäß Fig. 2 wird dieser Abschnitt 60 der Semitendinosus-Sehne dann in annähernd gleich lange Segmente 62, 64 unterteilt. Wie in Fig. 3 und 4 gezeigt, werden die Segmente 62, 64 sodann nebeneinander gelegt und mit Hilfe der Fäden 34 an ihren Enden verbunden. Anschließend werden die verbundenen Abschnitte 62, 64 zu der Schlaufe 20 gelegt, die bevorzugt noch mit Hilfe eines weiteren Fadens 66 an ihren Schenkeln 68 und 70 zusammengeschnürt wird. Schließlich wird der Faden 30 durch die Öffnung der Schlaufe 20 geführt, so daß der Kreuzbandersatz 16 dann mit Hilfe dieses Fadens 30 in der Sacklochbohrung 22 im Femur verspannt werden kann.

Die Platte 32, mit der dies, wie in Fig. 1 dargestellt, geschieht, ist gemäß Fig. 6 bzw. Fig. 7 hier eine längliche Platte mit insgesamt vier in gleichmäßigem Abstand voneinander angeordneten Löchern 74. Zum Verspannen des Kreuzbandersatzes 16 in der Sacklochbohrung 22 werden die Enden des Fadens 30 abwechselnd von oben bzw. von unten durch die Löcher 74 geführt und anschließend unter Zugspannung verknotet. Hierdurch bildet die Platte 32 einen Anker mit dem der Kreuzbandersatz 16 verspannt ist.

Die Platte 36 zum Verspannen des Kreuzbandersatzes 16 im proximalen Ende der Tibia 14 weist gemäß Fig. 8 und Fig. 9 eine runde, knopfartige Form auf. Sie besitzt vier im Quadrat angeordnete Löcher 76 sowie eine zentrisch angeordnete Erhebung 78. Die Erhebung 78 dient dazu, wie aus der Darstellung in Fig. 1 zu ersehen ist, die Platte 36 in bezug auf die Durchgangsbohrung 26 zu zentrieren. Hierdurch wird ein spielfreier Sitz der Platte 36 in bezug auf die Durchgangsbohrung 26 erreicht.

Die Platten 32 und 36 sind im vorliegenden Ausführungsbeispiel bevorzugterweise aus Titan. Anstelle der Platte 36 kann in einer alternativen Ausführung in bekannter Weise auch eine Schraube zum Fixieren des distalen Endes 24 des Kreuzbandersatzes 16 in der Tibia 14 verwendet werden.

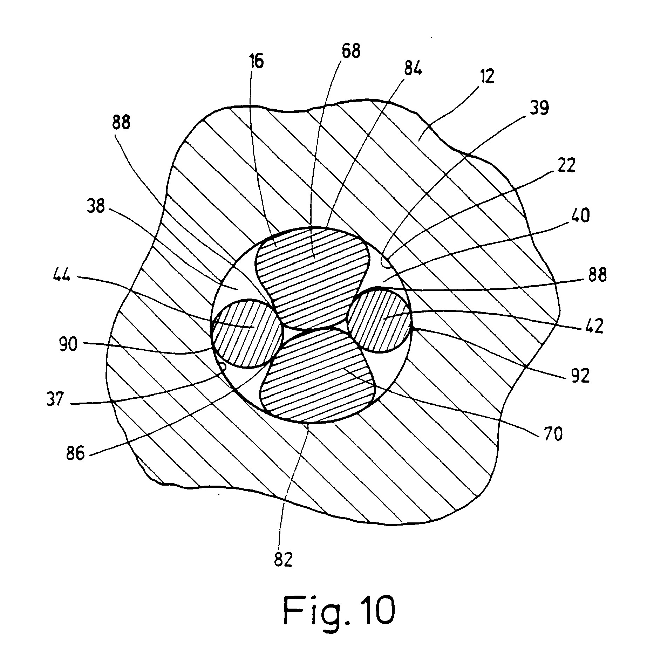

In der Querschnittsdarstellung der Fig. 10 ist erkennbar, wie durch das der Erfindung zugrunde liegende Einsetzen der Dübel 42, 44 in die Freiräume 38, 40 der Sitz des Kreuzbandersatzes 16 in der Sacklochbohrung 22 des Femur 12 verbessert wird. Der Durchmesser der Sacklochbohrung 22 ist so bemessen, daß er in etwa der Breite der Schlaufe 20 entspricht. Hierdurch ergeben sich bereits natürliche Kontaktstellen 82, 84 zwischen den Schenkeln 68, 70 der Schlaufe 20 und den Wänden 37, 39 der Sacklochbohrung 22. Dabei sind die Berührungsflächen der Kontaktstellen 82, 84 um so größer, je mehr der Kreuzbandersatz 16 in der Sacklochbohrung 22 zusammengedrückt wird.

Durch das der Erfindung zugrunde liegende Einsetzen der Dübel 42, 44 in die Freiräume 38, 40 wird nun einerseits erreicht, daß die Schenkel 68, 70 des Kreuzbandersatzes 16 noch weiter radial nach außen gedrückt werden. Hierdurch vergrößern sich bereits die Kontaktflächen der Kontaktstellen 82, 84. Darüber hinaus ergeben sich jedoch noch zusätzliche Kontaktstellen 86, 88 bzw. 90, 92, und zwar zwischen den eingesetzten Dübeln 42, 44 und den Schenkeln 68, 70 des Kreuzbandersatzes 16 sowie zwischen den Dübeln 42, 44 und den Wänden 37, 39 der Sacklochbohrung 22.

Da die Dübel 42, 44 in dem hier dargestellten Ausführungsbeispiel aus körpereigenem Knochenmaterial bestehen, wachsen sie an zusätzlichen Kontaktstellen 86, 88 bzw. 90, 92 sehr gut mit dem ebenfalls körpereigenen Material des Kreuzbandersatzes 16 und dem Femur zusammen. Dadurch ist der Halt des Kreuzbandersatzes 16 in der Sacklochbohrung 22 sowohl kurzfristig nach dem Einsetzen der Dübel 42, 44 als auch langfristig nach dem Zusammenwachsen der genannten Elemente um so größer, je größer die Kontaktflächen der Kontaktstellen 82, 84, 86, 88, 90, 92 sind.

Die Dübel 42, 44 erfüllen somit eine zweifache Funktion, nämlich einerseits den Kreuzbandersatz 16 in der Sacklochbohrung 22 bereits unmittelbar nach der Operation fester zu fixieren, und andererseits langfristig zusätzliche und größere Kontaktstellen zum Einwachsen des Kreuzbandersatzes 16 in der Sacklochbohrung 22 zu schaffen.

Vorteilhafterweise wird die Größe der Dübel 42, 44 bei dem der Erfindung zugrunde liegenden Verfahren der Größe der Freiräume 38, 40 angepaßt. Hieraus ergibt sich, daß sowohl die Länge als auch der Durchmesser der Dübel 42 und 44 verschieden sein kann.

Im folgenden wird nun ein bevorzugtes Ausführungsbeispiel eines erfindungsgemäßen Instrumentariums beschrieben, das, wie aufgrund der beengten Platzverhältnisse in einem Kniegelenk 10 leicht einzusehen ist, möglichst optimal an den zuvor beschriebenen Einsatzzweck angepaßt sein muß.

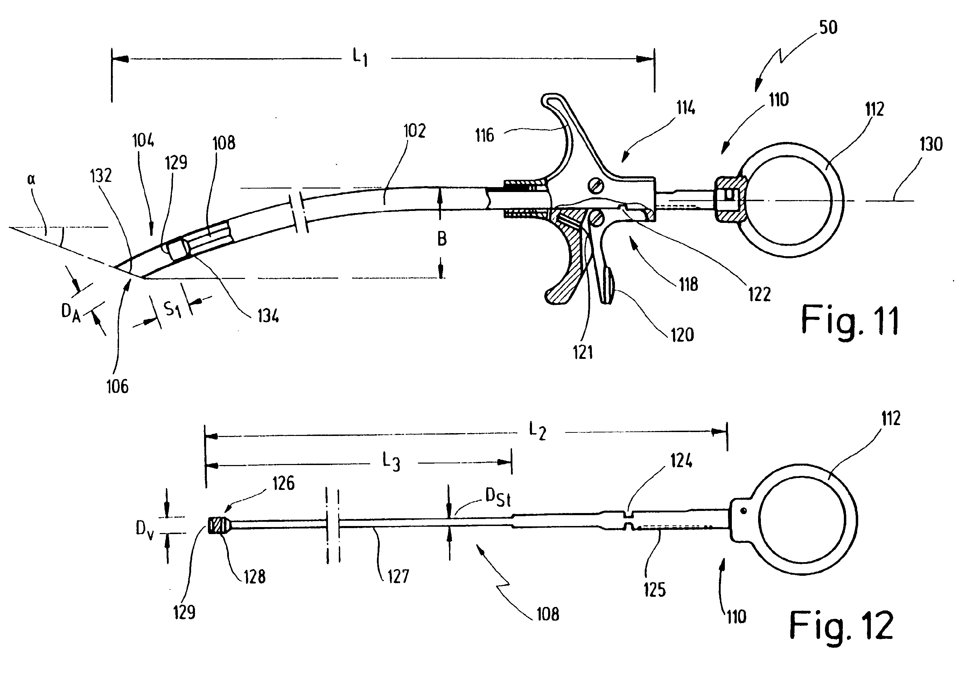

Der wichtigste Bestandteil des bevorzugten Instrumentariums ist ein Applikator, mit dem die Dübel 42, 44 an die Freiräume 38, 40 herangebracht und in diese eingesetzt werden können. Ein in bezug auf diese beiden Funktionen optimierter Applikator ist in der Figur 11 in seiner Gesamtheit mit der Bezugsziffer 50 bezeichnet.

Der Applikator 50 besitzt einen gekrümmten Hohlschaft 102, der an seinem distalen Ende 104 eine Öffnung 106 aufweist. Innerhalb des Hohlschaftes 102 befindet sich ein herausziehbarer Stößel 108 aus Kunststoff, der in seiner Gesamtheit in Fig. 12 dargestellt ist.

An seinem proximalen Ende 110 ist der Stößel 108 mit einem Griff 112 versehen, der im vorliegenden Fall ein Ringgriff ist. Das proximale Ende 114 des Hohlschaftes 102 weist demgegenüber einen Gegengriff 116 auf. In der Anwendung wird der Gegengriff 116 des Hohlschaftes 102 bevorzugt mit dem Zeige- und Mittelfinger einer Hand gehalten, während der Daumen derselben Hand zum Bedienen des Stößels 108 durch den Ringgriff 112 gesteckt wird. Hierdurch ist es möglich, den Applikator 50 mit einer Hand zu halten und gleichzeitig mit derselben Hand den Stößel 108 je nach Bedarf vorzuschieben und zurückzuziehen.

Der Applikator 50 weist des weiteren einen Verriegelungsmechanismus 118 auf. Der Verriegelungsmechanismus 118 beinhaltet einen verschwenkbaren Hebel 120, der durch eine an dem Gegengriff 116 abgestützte Feder 121 vorgespannt ist. An seinem verschwenkbaren Ende weist der Hebel 120 einen Zapfen 122 auf, der zum Verriegeln des Stößels 108 in eine dort angeordnete Ringnut 124 eingreift.

Im entriegelten Zustand des Verriegelungsmechanismus 118 kann der Stößel 108 vollständig aus dem Hohlschaft 102 nach hinten herausgezogen werden. Dies ist vor allem dann sinnvoll, wenn der Applikator 50 nach einem operativen Eingriff sterilisiert werden soll, da so eine gute Zugänglichkeit zu allen Teilen des Applikators 50 gegeben ist.

Umgekehrt kann der Stößel 108 nach dem Entriegeln des Verriegelungsmechanismus 118 in dem Hohlschaft 102 nach vorne geschoben werden. In diesem Fall gleitet der Zapfen 122 dann in eine im proximalen Endbereich des Stößels 108 angeordnete Längsnut 125.

Die Gesamtlänge L₁ des Hohlschaftes 102 des Applikators 50 beträgt im vorliegenden Ausführungsbeispiel 205 mm. Die Krümmung des Hohlschaftes 102 ist so bemessen, daß die Öffnung 106 um etwa 60 mm gegenüber dem proximalen Ende 114 seitlich versetzt liegt. Dies bedeutet, daß das in der Zeichnung angegebene Maß B etwa 60 mm beträgt.

Die Gesamtlänge L₂ des Stößels 108 gemessen vom distalen Ende 126 bis zu dem Anschlag des Griffs 112 beträgt im vorliegenden Ausführungsbeispiel 220 mm. Über diese Länge L₂ hinweg weist der Stößel 108 im wesentlichen drei Funktionsabschnitte auf.

Einen ersten dieser Funktionsabschnitte bildet das distale Ende 126, an das sich als zweiter Funktionsabschnitt ein Mittelabschnitt 127 anschließt. Das distale Ende 126 des Stößels 108 ist als Verdickung 128 ausgebildet, deren Außendurchmesser Dᵥ in etwa dem lichten Innendurchmesser D_{A} des Hohlschaftes 102 entspricht. Demgegenüber ist der Außendurchmesser D_{St} des Mittelabschnitts 127 geringer. Der dritte Funktionsabschnitt des Stößels 108 befindet sich am proximalen Ende 110. Er beinhaltet sowohl den Griff 112 als auch den Teil des Stößels 108, der zusammen mit dem Hebel 120 den Verriegelungsmechanismus 118 bildet.

Die Länge Länge L₃ des Mittelabschnitts 127 einschließlich der Verdickung 128 beträgt im vorliegenden Ausführungsbeispiel 160 mm. Der Durchmesser D_{St} des Mittelabschnitts 127 liegt bei etwa 3 mm. Der Durchmesser D_{A} des hier dargestellten Applikators 50 beträgt 5 mm. Dies Abmessungen haben sich für den angestrebten Verwendungszweck als gut geeignet erwiesen.

Die Verdickung 128 weist an ihrem distalen Ende eine Stoßfläche 129 auf, die bei dem hier dargestellten Ausführungsbeispiel konkav gewölbt ist. Hierdurch wird erreicht, daß sich ein in die Freiräume 38, 40 einzusetzender Dübel 42, 44 automatisch in bezug auf die Stoßfläche 129 zentriert.

Mit der Bezugsziffer 130 ist eine Bedienachse des Applikators 50 bezeichnet. Dies ist diejenige Achse, entlang der das proximale Ende 110 des Stößels 108 von einem Operateur verschoben werden kann, um einen Dübel 42, 44 in einen der Freiräume 38, 40 einzusetzen. Die Bedienachse 130 schließt mit dem Öffnungsquerschnitt 132 der Öffnung 106 des Hohlschaftes 102 einen Winkel α ein, der im hier dargestellten Ausführungsbeispiel etwa 20° beträgt. Je kleiner der Winkel α ist, desto stärker ist die Austrittsrichtung des Dübels 42, 44 aus der Öffnung 106 gegenüber der Bedienachse 130 versetzt. Hierdurch wird es dem Operateur erleichtert, den einzusetzenden Dübel 42, 44 durch eine von vorne zugängliche Öffnung im Kniegelenk 10 in die nach unten offene Sacklochbohrung 22 im Femur 12 einzusetzen.

In der in Fig. 11 dargestellten Betriebsstellung befindet sich der Stößel 108 innerhalb des Hohlschaftes 102 in einer ersten Position 134, in der sein distales Ende 126 vollständig in dem Hohlschaft 102 aufgenommen ist. Die Stoßfläche 129 des Stößels 108 befindet sich dabei in einem Abstand S₁ entfernt von der Öffnung 106 des Hohlschaftes 102. Gleichzeitig ist der Verriegelungsmechanismus 118 in dieser Betriebsstellung eingerastet, d.h. der Zapfen 122 greift in die Ringnut 124 des Stößels 108. Aufgrund des Abstandes S₁ ist der Applikator 50 so in der Lage, einen einzusetzenden Dübel 42, 44 in der Spitze des Hohlschaftes 102 weitgehend aufzunehmen. Hierdurch ist gewährleistet, daß der einzusetzende Dübel 42, 44 sicher und ohne das Risiko eines Verlierens an den Freiraum 38, 40 heran gebracht werden kann.

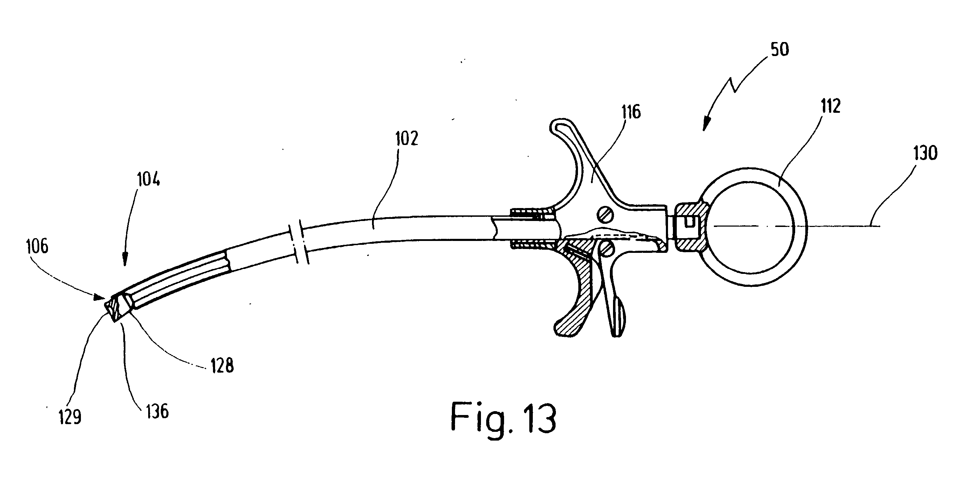

Demgegenüber befindet sich die Verdickung 128 des Stößels 108 in der in Fig. 13 dargestellten Betriebsstellung in einer zweiten Position 136, in der sie über die Öffnung 106 des Hohlschaftes 102 hinausragt. Dies ist der Fall, wenn der Stößel 108 so weit wie möglich in den Hohlschaft 102 des Applikators 50 eingeschoben ist. Der Zapfen 122 des Verriegelungsmechanismus 118 liegt in dieser Betriebsstellung am proximalen Ende der Längsnut 125. Dadurch, daß die Stoßfläche 129 des Stößels 108 in dieser Betriebsstellung über das distale Ende 104 des Hohlschaftes 102 hinausragt, ist es möglich, einen einzusetzenden Dübel 42, 44 vollständig aus dem Hohlschaft 102 heraus zuschieben und bereits mit Hilfe des Applikators 50 so weit in den Freiraum 38, 40 hineinzupressen, daß er beim Zurücknehmen des Applikators 50 nicht mehr von alleine herausfallen kann.

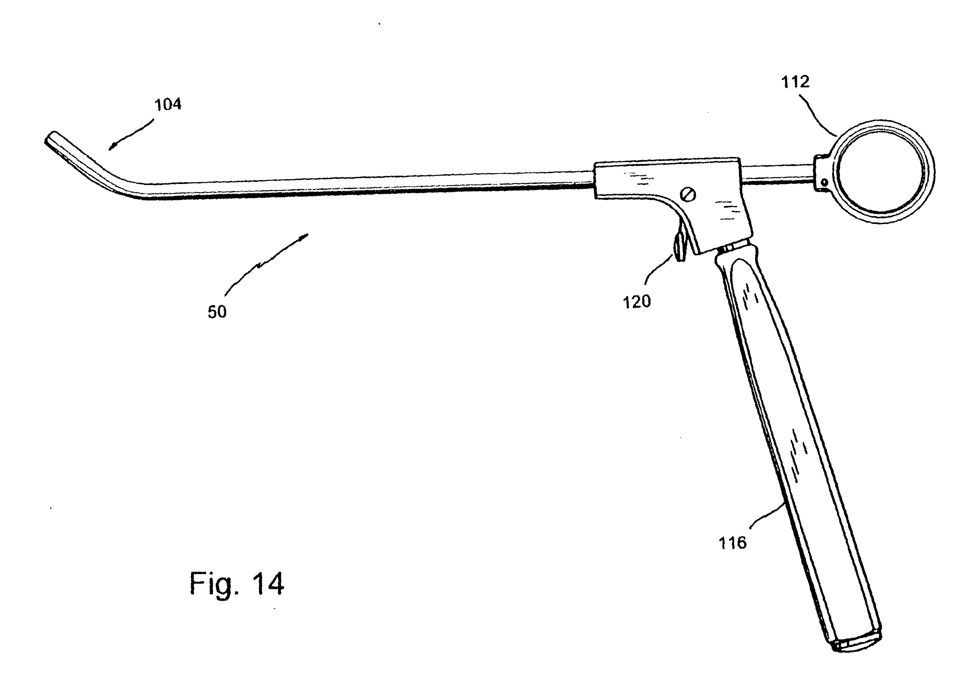

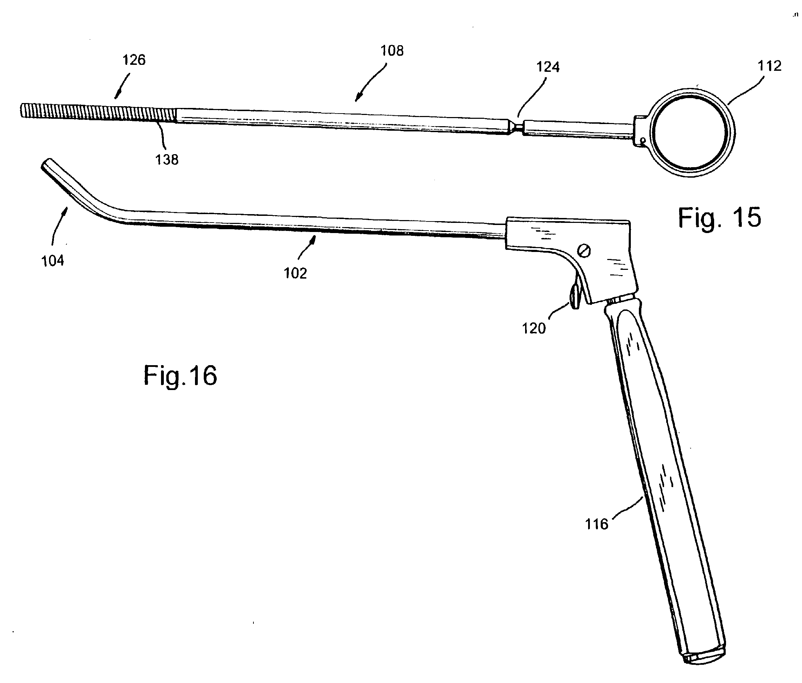

Die Figuren 14 bis 16 zeigen ein zweites bevorzugtes Ausführungsbeispiel des Applikators 50, wobei gleiche Bezugszeichen dieselben Elemente bezeichnen wie in den Figuren 11 bis 13. Bei diesem zweiten Ausführungsbeispiel ist der Hohlschaft 102 des Applikators 50 ausgehend von seinem proximalen Ende 114 zunächst gerade und nur in der Nähe seines distalen Endes 104 nach oben abgebogen. Des weiteren besteht der Stößel 108 hier aus Metall. An seinem distalen Ende 126 weist er eine spiralförmige, federartige Wicklung 138 aus Metall auf, die flexibel genug ist, sich dem abgebogenen distalen Ende 104 des Hohlschaftes 102 anzupassen.

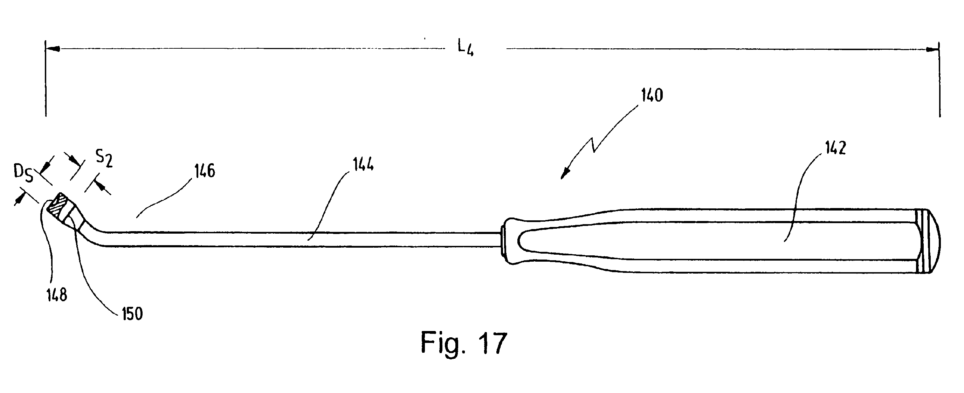

In Fig. 17 ist ein das erfindungsgemäße Instrumentarium ergänzender Stopfer in seiner Gesamtheit mit der Bezugsziffer 140 bezeichnet.

Der Stopfer 140 weist einen Griff 142 sowie einen stabilen, aus Metall gefertigten Schaft 144 auf. Seine Gesamtlänge L₄ beträgt in diesem Ausführungsbeispiel 275 mm.

Das distale Ende 146 des Stopfers 140 ist in einem Winkel von etwa 45° abgewinkelt und weist eine Stoßfläche 148 auf, die aus denselben Gründen wie die Stoßfläche 129 des Stößels 108 des Applikators 50 konkav gewölbt ist. Der Durchmesser Dₛ der Stoßfläche 148 beträgt bei dem hier dargestellten Stopfer 140 ebenfalls 5 mm. Vorzugsweise umfaßt das erfindungsgemäße Instrumentarium jedoch mehrere Stopfer 140, deren Enddurchmesser Dₛ voneinander verschieden sind. Hierdurch ist es dann möglich, zum Hineinpressen von Dübeln 42, 44 in unterschiedlich große Freiräume 38, 40 jeweils einen optimal angepaßten Stopfer 140 auszuwählen.

Der Stopfer 140 im vorliegenden Ausführungsbeispiel besitzt an seinem distalen Ende 146 eine Markierung 150, die sich in einem Abstand S₂ von der Stoßfläche 148 befindet. Mit Hilfe dieser Markierung 150 ist es dem Operateur möglich abzuschätzen, wie tief der einzusetzende Dübel 42, 44 bereits in den jeweiligen Freiraum 48, 40 hineingeschoben ist. Dies ermöglicht dem Operateur, zu entscheiden, ob er den Dübel 42, 44 im Einzelfall durch Aufbringen noch höherer Kräfte noch weiter in den Freiraum 38, 40 vorschieben muß.

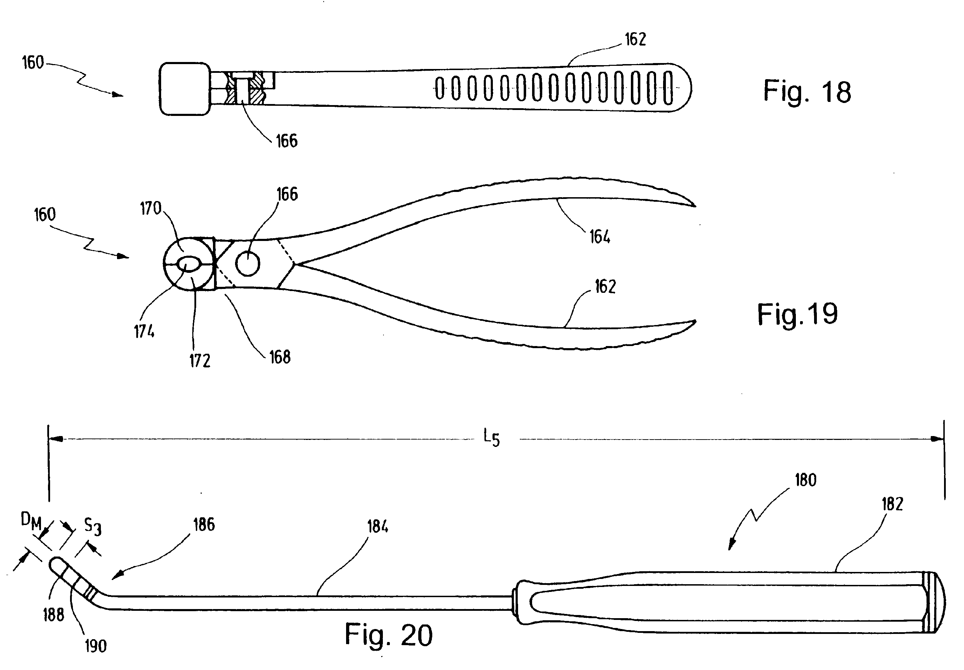

In den Figuren 18 und 19 ist eine das erfindungsgemäße Instrumentarium ergänzende Kompressionszange in ihrer Gesamtheit mit der Bezugsziffer 160 bezeichnet. Die Kompressionszange 160 besitzt zwei Griffhälften 162, 164, die durch einen Gelenkbolzen 166 in an sich bekannter Weise gegeneinander verschwenkbar verbunden sind. Jede der beiden Griffhälften 162, 164 weist an ihrem distalen Ende 168 eine Backe 170, 172 auf, die in aneinanderliegendem Zustand, d.h. bei geschlossener Kompressionszange 160, eine in diesem Fall ellipsenförmig-zylindrische Aussparung 174 einschließen. Alternative Ausführungsbeispiele der Kompressionszange 160 können jedoch auch kreisförmig-zylindrische oder konische Aussparungen 174 aufweisen.

Die Kompressionszange 160 dient bei dem der Erfindung zugrunde liegenden Verfahren dazu, aus Knochenmaterial, das mit Hilfe eines hier nicht dargestellten Hohlmeißels oder Kernbohrers beim Bohren der Sacklochbohrung 22 bzw. der Durchgangsbohrung 26 in Femur 12 bzw. Tibia 14 gewonnen werden kann, Dübel 42, 44 zu formen. Dazu wird das gewonnene Knochenmaterial, insbesondere der weiße Knochenschwamm (spongiosa) mit Hilfe der Kompressionszange 160 in die gewünschte Form gepreßt.

In Fig. 20 ist ein das erfindungsgemäße Instrumentarium ergänzender Meßstab in seiner Gesamtheit mit der Bezugsziffer 180 bezeichnet.

Der Meßstab 180 weist einen Griff 182 sowie einen stabilen, aus Metall gefertigten Schaft 184 auf. Seine Gesamtlänge L₅ beträgt wiederum 275 mm. Das distale Ende 186 des Meßstabes 180 entspricht in etwa der Form des einzusetzenden Dübels 42, 44 und ist ebenso wie das distale Ende 146 des Stopfers 140 in einem Winkel von etwa 45° abgebogen. Hierdurch wird dem Operateur das Einführen des distalen Endes 186 in den Freiraum 38, 40 zum Bestimmen der Größe dieses Freiraums erleichtert. Um insbesondere den Kreuzbandersatz 16 beim Bestimmen der Größe des Freiraums 38, 40 nicht zu beschädigen, ist das distale Ende 186 des Meßstabes 180 gleichmäßig abgerundet.

Der Enddurchmesser D_{M} des distalen Endes 186 des Meßstabes 180 beträgt hier 4 mm. Wie bereits eingangs angedeutet, beinhaltet das erfindungsgemäße Instrumentarium jedoch mehrere Meßstäbe 180, deren Enddurchmesser D_{M} voneinander verschieden sind. Durch Ausprobieren verschiedener Meßstäbe ist es dem Operateur damit möglich, auf einfache Weise die Größe des Freiraums 38, 40 und damit die erforderliche Größe eines einzusetzenden Dübels 42, 44 zu bestimmen.

Der Meßstab 180 des vorliegenden Ausführungsbeispiels weist an seinem distalen Ende insgesamt drei Markierungen 188, 190 auf, anhand derer der Operateur erkennen kann, wie tief der jeweilige Freiraum 38, 40 innerhalb der Sacklochbohrung 22 reicht. Dazu befinden sich die Markierungen 188, 190 in vorbekannten Abständen S₃ zum Ende des Meßstabes 180.

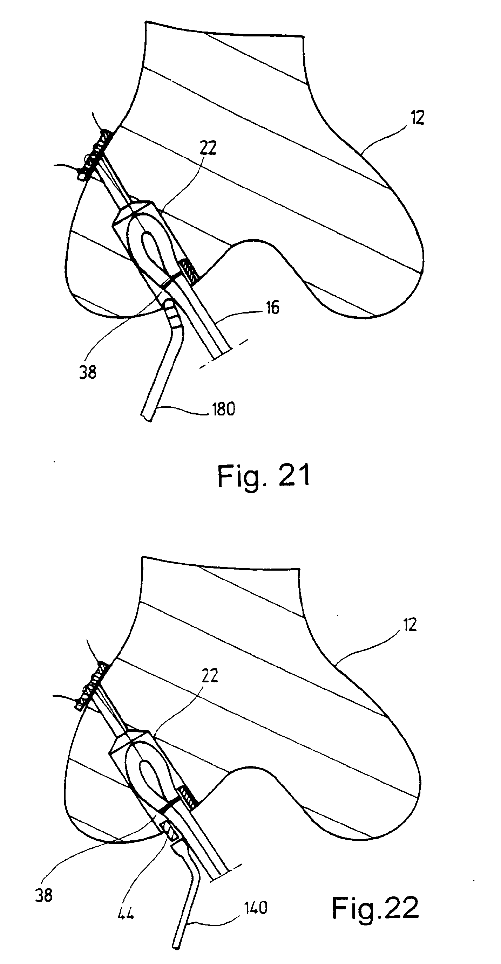

In Fig. 21 ist in einer der Fig. 1 entsprechenden Darstellung die Verwendung des Meßstabes 180 zur Bestimmung der Größe des Freiraums 138 dargestellt. Wie hier zu erkennen ist, wird das distale Ende 186 des Meßstabes 180 unter Aufbringung einer Druckkraft so weit wie möglich in den Freiraum 38 vorgeschoben. Diese Prozedur wiederholt der Operateur gegebenenfalls mit mehreren Meßstäben unterschiedlichen Enddurchmessers D_{M}.

Zusätzlich zu seiner Funktion als Meßmittel wirkt der Meßstab 180 in diesem Fall auch als Dilatator, d.h. er dient gleichzeitig zum Aufweiten des Freiraums 38. Hierdurch ist es dem Operateur möglich, die Größe des Freiraums 38 unter Berücksichtigung der sich beim Einsetzen des Dübels 42, 44 ergebenden Aufweitung zu bestimmen.

In Fig. 22 ist in einer der Fig. 1 entsprechenden Darstellung die Verwendung des Stopfers 140 gezeigt. Der Stopfer 140 wird im Anschluß an das eigentliche Einsetzen des Dübels 44 benutzt, um den Dübel 44 so tief wie möglich in den Freiraum 38 hineinzupressen.

Insgesamt beinhaltet das der Erfindung zugrunde liegende Verfahren somit folgende Schritte:
- Herstellen eines Kreuzbandersatzes 16, vorzugsweise mit Hilfe eines zu einer Schlaufe 20 gelegten Abschnitts der Semitendinosus-Sehne,
- Bohren einer Sacklochbohrung 22 im distalen Ende des Femur 12 sowie einer Durchgangsbohrung 26 im proximalen Ende der Tibia,
- Einsetzen und Verspannen des Kreuzbandersatzes 16 in der Sacklochbohrung 22 sowie der Durchgangsbohrung 26 mit Hilfe von Ankermitteln 30, 32 bzw. 34, 36,
- Bestimmen der Größe der Freiräume 38, 40 zwischen dem Kreuzbandersatz 16 und den Wänden 37, 39 der Sacklochbohrung 22,
- Formen eines oder mehrerer einzusetzender Dübel 42, 44, vorzugsweise aus dem beim Anbringen der Sacklochbohrung 22 bzw. der Durchgangsbohrung 26 gewonnenen Knochenschwamm,
- Einsetzen des Dübels 42, 44 in den Freiraum 38, 40 und
- weiteres Hineinpressen des Dübels 42, 44 in den Freiraum 38, 40.

Das der Erfindung zugrunde liegende Verfahren ist besonders vorteilhaft, wenn als Kreuzbandersatz 16, wie beschrieben, ein Abschnitt der Semitendinosus-Sehne verwendet wird. Der Grund hierfür ist, daß vor allem in diesem Fall der Kreuzbandersatz 16 in Form der Schlaufe 20 gelegt wird.

Das Einsetzen von Dübeln in vorhandene Freiräume und dementsprechend natürlich auch das erfindungsgemäße Instrumentarium kann jedoch ebenso auch verwendet werden, wenn als Kreuzbandersatz 16 ein Abschnitt der Patellar-Sehne oder eine synthetische Kreuzbandplastik verwendet wird. Es versteht sich außerdem, daß grundsätzlich auch Dübel aus synthetischem Material in die genannten Freiräume 38, 40 einsetzbar sind.

## Patentansprüche

1. Instrumentarium zum Implantieren eines Kreuzbandersatzes (16) in einem Kniegelenk, mit einem Applikator (50) zum Einsetzen eines Dübels (42, 44) in eine Bohrung (22), in der der Kreuzbandersatz (16) aufgenommen ist, und zwar in einen Freiraum (38, 40) zwischen dem Kreuzbandersatz (16) und einer Wand (37, 39) der Bohrung (22), an der der Kreuzbandersatz (16) fixiert werden soll, wobei der Applikator (50) einen an seinem distalen Ende (104) offenen Hohlschaft (102) sowie einen in dem Hohlschaft (102) geführten Stößel (108) aufweist und wobei der Applikator (50) ein Bedienelement (112, 116) besitzt, mit dem der Stößel (108) derart in dem Hohlschaft (102) verschiebbar ist, daß ein in den Hohlschaft (102) eingebrachter Dübel (42, 44) durch die Öffnung (106) an dem distalen Ende (104) des Hohlschaftes (102) ausstoßbar ist, wobei der Applikator (50) einen lösbare Verriegelungsmechanismus (118) zum Feststellen des Stößels (108) relativ zu dem Hohlschaft (102) aufweist, wobei das Instrumentarium außerdem mehrere Meßstäbe (180) zum Bestimmen der Größe des Freiraums (38, 40) aufweist, wobei das distale Ende (186) jedes Meßstabes (180) in etwa der Form des einzusetzenden Dübels (42, 44) entspricht und wobei die Enddurchmesser D_{M} der Meßstäbe (180) voneinander verschieden sind, und es weiterhin zumindest einen Stopfer (140) beinhaltet, mit dem der Dübel (42, 44) in den Freiraum (38, 40) hineinpreßbar ist.

2. Instrumentarium nach Anspruch 1, **dadurch gekennzeichnet, daß** der Hohlschaft (102) gekrümmt ist.

3. Instrumentarium nach Anspruch 2, **dadurch gekennzeichnet, daß** der Hohlschaft (102) über seine Länge gleichmäßig gekrümmt ist.

4. Instrumentarium nach Anspruch 2, **dadurch gekennzeichnet, daß** der Hohlschaft (102) entlang zumindest seines Mittelabschnitts im wesentlichen gerade ist und daß er in der Nähe seines distalen Endes in eine Richtung abgebogen ist.

5. Instrumentarium nach Anspruch 1, **dadurch gekennzeichnet, daß** der Hohlschaft (102) durchgehend gerade ist.

6. Instrumentarium nach einem der Ansprüche 1 bis 5, **dadurch gekennzeichnet, daß** der Stößel (108) eine Verdickung (128) an seinem distalen Ende (126) sowie einen Mittelabschnitt (127) der Länge L₃ aufweist, wobei der Außendurchmesser D_{V} der Verdickung (128) dem Innendurchmesser D_{A} des Hohlschaftes (102) entspricht und wobei der Außendurchmesser D_{St} des Mittelabschnitts (127) geringer ist als der Außendurchmesser D_{V} der Verdickung (128).

7. Instrumentarium nach einem der Ansprüche 1 bis 6, **dadurch gekennzeichnet, daß** das distale Ende (126) des Stößels (108) zwischen einer ersten (134) und einer zweiten (136) Position verschiebbar ist, wobei sich das distale Ende (126) in der ersten Position (134) vollständig innerhalb des Hohlschaftes (102) befindet, und zwar in einem Abstand S₁ zu der Öffnung (106) des Hohlschaftes (102), und wobei das distale Ende (126) in der zweiten Position (136) über die Öffnung (106) des Hohlschaftes (102) hinausragt.

8. Instrumentarium nach einem der Ansprüche 1 bis 7, **dadurch gekennzeichnet, daß** das Bedienelement (112, 116) entlang einer Bedienachse (130) bedienbar ist und daß der Öffnungsquerschnitt (132) der Öffnung (106) des Hohlschaftes (102) in bezug auf die Bedienachse (130) in einem Winkel α von weniger als 90°, vorzugsweise zwischen 0° und 45° angeordnet ist.

9. Instrumentarium nach einem der Ansprüche 1 bis 8, **dadurch gekennzeichnet, daß** das Bedienelement (112, 116) des Applikators (50) einen Griff (112) am proximalen Ende (110) des Stößels (108), vorzugsweise einen mit dem Daumen bedienbaren Ringgriff (112), sowie einen Gegengriff (116) an dem Hohlschaft (102) aufweist.

10. Instrumentarium nach einem der Ansprüche 1 bis 9, **dadurch gekennzeichnet, daß** der Stößel (108) vollständig aus dem Hohlschaft (102) herausziehbar ist.

11. Instrumentarium nach Anspruch 1, **dadurch gekennzeichnet, daß** der Verriegelungsmechanismus (118) einen mit einer Feder (121) vorgespannten Hebel (120) aufweist, an dessen Ende ein Zapfen (122) angeordnet ist, der im verriegelten Zustand in eine Ringnut (124) des Stößels (108) greift.

12. Instrumentarium nach Anspruch 1, **dadurch gekennzeichnet, daß** jeder Meßstab (180) an seinem distalen Ende (186) zumindest eine sichtbare Markierung (188, 190) aufweist, die in einem vorbestimmten Abstand S₃ zum Ende des Meßstabes (180) angeordnet ist.

13. Instrumentarium nach Anspruch 1, **dadurch gekennzeichnet, daß** der Stopfer (140) an seinem distalen Ende (146) eine konkav gewölbte Stoßfläche (148) aufweist.

14. Instrumentarium nach Anspruch 13, **dadurch gekennzeichnet, daß** der Stopfer (140) an seinem distalen Ende (146) in einer vorbestimmten Entfernung S₂ zur Stoßfläche (148) eine sichtbare Markierung (150) aufweist.

15. Instrumentarium nach Anspruch 13, **dadurch gekennzeichnet, daß** es mehrere Stopfer (140) beinhaltet, deren Enddurchmesser D_{S} voneinander verschieden sind.

## Claims

1. Instrumentarium for implanting a crucial ligament replacement (16) in a knee-joint, with an applicator (50) for inserting a fastener (42, 44) in a bore (22) in which the crucial ligament replacement (16) is received, specifically in a clear space (38, 40) between the crucial ligament replacement (16) and a wall (37, 39) of the bore (22), to which the crucial ligament replacement (16) is to be fixed, where said applicator (50) comprises a hollow shaft (102) that is open at its distal end (104) and a push-rod (108) guided in said hollow shaft (102), and where said applicator (50) comprises an operating element (112, 116) by means of which said push-rod (108) can be displaced in said hollow shaft (102) so that a fastener (42, 44), previously introduced into said hollow shaft (102), can be pushed out through an opening (106) at the distal end (104) of said hollow shaft (102), wherein said applicator (50) comprises a releasable locking mechanism (118) for fixing said push-rod (108) relative to said hollow shaft (102), wherein said instrumentarium further comprises a plurality of scales (180) for determining the size of said clear space (38, 40), the distal end (186) of each scale (180) corresponds substantially to the shape of said fastener (42, 44) to be inserted and the end diameters D_{M} of the scales (180) being different one from another, and wherein it further comprises at least one tamping tool (140) by means of which said fastener (42, 44) can be pressed into said clear space (38, 40).

2. Instrumentarium of claim 1, **characterized in that** the hollow shaft (102) is curved.

3. Instrumentarium of claim 2, **characterized in that** the hollow shaft (102) is curved regularly over its length.

4. Instrumentarium of claim 2, **characterized in that** the hollow shaft (102) is substantially straight at least over its central portion and is then bent off in one direction near its distal end.

5. Instrumentarium of claim 1, **characterized in that** the hollow shaft (102) is straight over its full length.

6. Instrumentarium of anyone of claims 1 through 5, **characterized in that** the push-rod (108) comprises a thicker portion (128) at its distal end (126) and a central portion (127) of a length L₃, the outer diameter D_{V} of said thicker portion (128) corresponds to the inner diameter D_{A} of the hollow shaft (102), and **in that** the outer diameter D_{St} of the central portion (127) is smaller than the outer diameter D_{V} of said thicker portion (128).

7. Instrumentarium of anyone of claims 1 through 6, **characterized in that** the distal end (126) of said push-rod (108) can be displaced between a first (134) and a second (136) position, with that distal end (126) being fully positioned in said hollow shaft (102) in the first position (134), i.e. at a distance S₁ from said opening (106) of said hollow shaft, and **in that** the distal (126) projects beyond said opening (106) of said hollow shaft (102) in its second position (136).

8. Instrumentarium of anyone of claims 1 through 7, **characterized in that** the operating element (112, 116) can be operated along an operating axis (130), and **in that** the opening cross section (132) of the opening (106) of the hollow shaft (102) is arranged at an angle α of less than 90°, preferably between 0° and 45°, related to the operating axis (130).

9. Instrumentarium of anyone of claims 1 through 8, **characterized in that** the operating element (112, 116) of the applicator (50) comprises a handle (112) at the proximal end (110) of the push-rod (108), preferably a ring (112) that can be actuated by the thumb, and a counter-handle (116) on the hollow shaft (102).

10. Instrumentarium of anyone of claims 1 through 9, **characterized in that** the push-rod (108) can be fully withdrawn from the hollow shaft (102).

11. Instrumentarium of claim 1, **characterized in that** the locking mechanism (118) comprises a lever (120), biased by a spring (121), whose end carries a lug (122) that engages an angular groove (164) in said push-rod (118) in the locked condition.

12. Instrumentarium of claim 1, **characterized in that** each scale (180) is provided at its distal end (186) with at least one visible mark (188, 190), arranged at a certain distance S₃ from the end of the scale (180).

13. Instrumentarium of claim 1, **characterized in that** the tamping tool (140) is provided with a dished impact surface (148) at its distal end (146).

14. Instrumentarium of claim 13, **characterized in that** the tamping tool (140) is provided with a visible mark (150) at its distal end (146) at a given distance S₂ from said impact surface (148).

15. Instrumentarium of claim 13, **characterized in that** a plurality of tamping tools (140) with different end diameters D_{S}.

## Revendications

1. Instruments chirurgicaux pour l'implantation d'un ligament croisé de remplacement (16) dans une articulation du genou, avec un applicateur (50) pour l'insertion d'une cheville (42, 44) dans un orifice (22) dans lequel est logé le ligament croisé de remplacement (16), à savoir dans un espace libre (38, 40) entre le ligament croisé de remplacement (16) et une paroi (37, 39) de l'orifice (22) sur laquelle doit être fixé le ligament croisé de remplacement (16), l'applicateur (50) présentant un tube creux ouvert (102) à son extrémité distale (104) ainsi qu'une tige (108) guidée dans le tube creux (102) et l'applicateur (50) possédant un élément de commande (112, 116) à l'aide duquel la tige (108) peut être déplacée dans le tube creux (102) de manière à ce qu'une cheville (42, 44) insérée dans le tube creux (102) puisse être repoussée à l'extrémité distale (104) du tube creux (102) par l'ouverture (106), l'applicateur (50) comprenant un mécanisme de verrouillage (118) détachable pour bloquer la tige (108) de manière relative au tube creux (102), les instruments chirurgicaux comportant par ailleurs plusieurs règles graduées (180) pour déterminer la dimension de l'espace libre (38, 40), l'extrémité distale (186) de chaque règle graduée (180) correspondant à peu près à la forme de la cheville (42, 44) devant être insérée, le diamètre d'extrémité Dₓ des règles graduées (180) étant différent l'un de l'autre et lesdits instruments chirurgicaux contenant de plus, au moins un pousseur (140) à l'aide duquel la cheville (42, 44) peut être enfoncée à l'intérieur de l'espace libre (38, 40).

2. Instruments chirurgicaux selon la revendication 1, **caractérisés en ce que** le tube creux (102) est cintré.

3. Instruments chirurgicaux selon la revendication 2, **caractérisés en ce que** le tube creux (102) est cintré régulièrement sur sa longueur.

4. Instruments chirurgicaux selon la revendication 2, **caractérisés en ce que** le tube creux (102) est sensiblement droit, au moins le long de sa section centrale et qu'il est courbé dans un sens près de son extrémité distale.

5. Instruments chirurgicaux selon la revendication 1, **caractérisés en ce que** le tube creux (102) est droit de bout en bout.

6. Instruments chirurgicaux selon l'une quelconque des revendications 1 à 5, **caractérisés en ce que** la tige (108) comporte un épaississement (128) à son extrémité distale (126) ainsi qu'une section centrale (127) d'une longueur L₃, le diamètre extérieur Dᵥ de l'épaississement (128) correspondant au diamètre intérieur D_{A} du tube creux (102) et le diamètre extérieur D_{St} de la section centrale (127) étant plus petit que le diamètre extérieur Dᵥ de l'épaississement (128).

7. Instruments chirurgicaux selon l'une quelconque des revendications 1 à 6, **caractérisés en ce que** l'extrémité distale (126) de la tige (108) peut être déplacée entre une première (134) et une seconde position (136), l'extrémité distale (126) se trouvant entièrement à l'intérieur du tube creux (102) dans la première position (134), et notamment à une distance S₁ de l'ouverture (106) du tube creux (102), et l'extrémité distale (126) saillant de l'ouverture (106) du tube creux (102) dans la seconde position (136).

8. Instruments chirurgicaux selon l'une quelconque des revendications 1 à 7, **caractérisés en ce que** l'élément de commande (112, 116) peut être manoeuvré le long d'un axe de commande (130) et que la section d'ouverture (132) de l'ouverture (106) du tube creux (102) forme avec l'axe de commande (130), un angle α inférieur à 90°, de préférence entre 0° et 45°.

9. Instruments chirurgicaux selon l'une quelconque des revendications 1 à 8, **caractérisés en ce que** l'élément de commande (112, 116) de l'applicateur (50) comporte une poignée (112) à l'extrémité proximale (110) de la tige (108), de préférence une poignée annulaire (112) manoeuvrable avec le pouce, ainsi qu'une contre-poignée (116) sur le tube creux (102).

10. Instruments chirurgicaux selon l'une quelconque des revendications 1 à 9, **caractérisés en ce que** la tige (108) est entièrement extractible du tube creux (102).

11. Instruments chirurgicaux selon la revendication 1, **caractérisés en ce que** le mécanisme de verrouillage (118) présente un levier (120) précontraint par un ressort (121), à l'extrémité duquel est disposé un tenon (122) qui s'engage dans une rainure annulaire (124) de la tige (108) à l'état verrouillé.

12. Instruments chirurgicaux selon la revendication 1, **caractérisés en ce que** chaque règle graduée (180) présente à son extrémité distale (186) au moins un marquage visible (188, 190) qui est disposé à une distance S₃ déterminée de l'extrémité de la règle graduée (180).

13. Instruments chirurgicaux selon la revendication 1, **caractérisés en ce que** le pousseur (140) comporte à son extrémité distale (146), une surface de choc (148) concave cintrée.

14. Instruments chirurgicaux selon la revendication 13, **caractérisés en ce que** le pousseur (140) présente à son extrémité distale (146), un marquage visible (150) qui est disposé à une distance S₂ déterminée de la surface de choc (148).

15. Instruments chirurgicaux selon la revendication 13, **caractérisés en ce qu'**ils contiennent plusieurs pousseurs (140) dont les diamètres d'extrémité Dₛ sont différents l'un de l'autre.
